# EUROPÄISCHE PATENTANMELDUNG

(11) **EP 3 786 609 A1**
(43) Veröffentlichungstag der Anmeldung: **03.03.2021**
(21) Anmeldenummer: 20191528.7
(22) Anmeldetag: 18.08.2020
(51) Int. Cl.: G01N 3/02, G01N 1/28, B05D 1/40, B29C 41/12, G01N 3/08, G01N 33/32

(54) **VERFAHREN ZUR ERSTELLUNG VON FREISTEHENDEN, DÜNNEN SCHICHTEN AUS WANDFARBE**

(30) Priorität: 27.08.2019 AT 507382019
(71) Anmelder: Technische Universität Wien, 1040 Wien (AT)
(72) Erfinder: Radoevski, Aleksandar, 1120 Wien (AT)
(74) Vertreter: Patentanwaltskanzlei Matschnig & Forsthuber OG

(57) **Zusammenfassung**

Ein erfindungsgemäßes Verfahren zur Herstellung einer freistehenden dünnen Schicht aus Wandfarbe umfasst die folgende Herstellungsschritte:
-a- Bereitstellen einer saugfähigen Unterlagsplatte (10) zum Auftrag von Wandfarbe (20);
-b- Auftragen von Wandfarbe (20) auf der Unterlagsplatte (10);
-c- Glätten der auf der Unterlagsplatte (10) aufgetragenen Wandfarbe (20) mithilfe eines Glättwerkzeugs (30) zu einer gleichmäßigen Wandfarbschicht (22) mit einer definierten Schichtstärke (21);
-d- Trocknen der Wandfarbschicht (22) auf der Unterlagsplatte (10);
-e- Einlegen der saugfähigen Unterlagsplatte (10) in ein Wasserbad (40), wobei die Oberseite (12) der Unterlagsplatte (10) mitsamt der darauf befindlichen getrockneten Wandfarbschicht (22) aus dem Wasserbad (40) herausragt;
-f- Entnehmen der Unterlagsplatte (10) aus dem Wasserbad (40);
-g- Pressen (50) und gleichzeitiges Trocknen (60) der Wandfarbschicht (22);
-h- Abheben der getrockneten und gepressten Wandfarbschicht (22) von der Unterlagsplatte (10) mit einem Abhebewerkzeug (34).

## Beschreibung

Die Erfindung betrifft ein neuartiges Verfahren zur Herstellung von freistehenden, dünnen Schichten aus Wandfarbe, die als Probekörper einer anschließenden Materialprüfung unterzogen werden können.

EU-weit gibt es derzeit noch keine normierten Prüfmethoden, die für die zuverlässige und reproduzierbare Bestimmung der Dehnfähigkeit von Wandfarben geeignet sind. Etablierte Prüfmethoden, die für ähnliche Anwendungen approbiert sind und derzeit behelfsweise zur Bestimmung der Dehnfähigkeit von Wandfarben herangezogen werden müssen, bringen jedoch hinsichtlich der jeweils vorgeschriebenen Prüfvorrichtungen und/oder der in den Methodenvorschriften jeweils vorgegebenen Probenform deutliche Einschränkungen mit sich. So sind beispielsweise die Prüfmethoden zur Bestimmung des Rissüberbrückungsvermögens von Beschichtungsstoffen und -systemen für mineralische Substrate und Beton im Außenbereich nach DIN EN 1062-7 und die Prüfmethoden zur Bestimmung des Rissüberbrückungsvermögens von kunststoffmodifizierten Bitumendickbeschichtungen zur Bauwerksabdichtung nach DIN EN 15812 für die Untersuchung von Wandfarben als Innenraumbeschichtungen ungeeignet, da solche Wandfarben für die Innenraumanwendung alleine schon den durch die Prüfvorrichtungen der vorgenannten Prüfmethoden verursachten Belastungen nicht standhalten können. Mit diesen Prüfmethoden können lediglich bestimmte Fassadenfarben getestet werden, wenn diese eine für die jeweilige Prüfvorrichtung ausreichende Schichtdicke und entsprechende Festigkeit aufweisen.

Des Weiteren wird derzeit zur Bestimmung der Dehnfähigkeit von Wandfarben der Zugversuch nach DIN EN ISO 527-1 eingesetzt, wobei dieses Prüfverfahren für die Bestimmung der Zugeigenschaften von Kunststoffen entwickelt wurde, wobei Zugproben bestimmter Form und Dimensionen nach DIN EN ISO 527-2 erforderlich sind. Die Herstellung solcher Proben erfolgt derzeit allerdings mittels eines Trägermaterials, auf das die Wandfarbe aufgetragen wird und mit diesem dauerhaft in Verbund bleibt. Daraus entsteht jedoch in nachteiliger Weise eine Kompositprobe, bei der die Eigenschaften des Trägermaterials Einfluss auf das Messergebnis nehmen können. Zur Erstellung von Zugproben, die nur aus der Wandfarbe selbst bestehen, wird derzeit ein Verfahren eingesetzt, bei dem die Wandfarbe auf eine mit Teflonfolie überzogene, ebene Oberfläche beispielsweise mithilfe einer Rakelmaschine aufgetragen wird. Nach Austrocknung der aufgetragenen Schicht lässt sich diese von der Folie entfernen und es können anschließend daraus Zugproben in gewünschter Form und mit den jeweils gewünschten Abmessungen ausgeschnitten werden. Dieses Verfahren eignet sich jedoch nur eingeschränkt bei gewissen Wandfarben bzw. Fassadenfarben, deren Trocknungsverhalten keine Saugfähigkeit des Untergrundes erfordert. Alle derzeit bekannten Innenraumbeschichtungen und viele Fassadenfarben, die für den Auftrag auf einem saugfähigen Untergrund entwickelt sind, scheiden damit aus und können mit diesem derzeit verwendeten Prüfverfahren nicht auf deren Dehnfähigkeit hin untersucht werden.

Aus dem Stand der Technik sind bereits einige Verfahren bekannt, um freistehende, dünne Materialschichten herstellen zu können, die dann beispielsweise auf deren Materialeigenschaften wie deren Dehnfähigkeit, Biegefestigkeit und/oder Zugfestigkeit überprüft werden können.

Beispielsweise ist aus dem Dokument US 2016/0339609 ein Verfahren bekannt geworden, um dünne, poröse Schichten aus einem Polymer mit Hilfe einer zusätzlichen Schicht eines wasserlöslichen Polymers herzustellen. Die zusätzliche wasserlösliche Polymerschicht wird dabei zwischen dem Untergrund (dem Substrat) und der zu erstellenden Schicht aufgetragen. Durch Auflösen dieser zusätzlichen, wasserlöslichen Zwischenschicht in einem Wasserbad kann die zu erstellende, poröse Polymerschicht vom Untergrund getrennt werden. Dabei ist jedoch der Einsatz von Alkohol und Lösungsmittel im Wasserbad erforderlich, um das vollständige Auflösen der Zwischenschicht zu gewährleisten. Für die Herstellung von dünnen Schichten aus Wandfarbe ist dieses Verfahren daher nicht geeignet.

Des Weiteren ist aus dem Dokument DE 10305439 A1 ein Verfahren bekannt geworden, mit dem dünne Schichten aus Acrylat mithilfe eines antihaftenden Filmmaterials, welches als Untergrund dient, hergestellt werden können. Nach dem Auftragen der Acrylat-Schicht auf dem Filmmaterial muss die Acrylat-Schicht getrocknet werden. Dieses Verfahren ist jedoch für übliche Wandfarben nicht geeignet, da durch den Einsatz eines antihaftenden Untergrundes und einer erforderlichen, zwangsweisen Trocknung eine Rissbildung und Verzerrung einer Wandfarbenschicht nicht zu vermeiden wäre.

Beispielsweise ist aus dem Dokument US 2002004126 A1 die Herstellung von dünnen Schichten eines organischen Materials als Beschichtungsmaterial bekannt geworden, wobei diese Beschichtung auf einem hydrophoben Untergrund erfolgt. Dieser hydrophobe Untergrund ermöglicht nach dem Trocknen der aufgetragenen Beschichtung eine Trennung der bereits erhärteten Schicht vom Untergrund durch Abziehen der Schicht. Dieses Verfahren ist auf Wandfarben nicht übertragbar, da die derzeit bekannten, herkömmlichen Wandfarben, insbesondere Innenwandfarben, nicht für das Auftragen auf hydrophoben, wasserabweisenden Untergründen entwickelt sind. Daher ist aufgrund der Eigenschaften des in der US 2002004126 A1 genannten hydrophoben Untergrundes mit einer antihaftenden Oberfläche sowie einer für Wandfarben unzureichenden Saugfähigkeit von einer unerwünschten Rissbildung und Verzerrung einer Wandfarbschicht während der Trocknung der Wandfarbe auszugehen.

Die Erfindung stellt sich die Aufgabe, ein neues Verfahren zur Herstellung von freistehenden, dünnen Schichten aus Wandfarbe anzugeben, das die aus dem Stand der Technik bekannten Nachteile der bisherigen Verfahren überwindet.

Diese Aufgabe wird vom erfindungsgemäßen Herstellungsverfahren mit den Merkmalen des Anspruchs 1 gelöst. Die Unteransprüche betreffen weitere besonders vorteilhafte Ausgestaltungen der Erfindung, die auch in der Beschreibung dargelegt sind.

Das erfindungsgemäße Verfahren zur Herstellung einer freistehenden dünnen Schicht aus Wandfarbe umfasst die folgenden Herstellungsschritte:
- a- Bereitstellen einer saugfähigen Unterlagsplatte zum Auftrag von Wandfarbe;
- b- Auftragen von Wandfarbe auf der Oberseite der saugfähigen Unterlagsplatte;
- c- Glätten der auf der Unterlagsplatte aufgetragenen Wandfarbe mithilfe eines Glättwerkzeugs zu einer gleichmäßigen Wandfarbschicht mit einer definierten Schichtstärke;
- d- Trocknen der Wandfarbschicht auf der Unterlagsplatte;
- e- Einlegen der saugfähigen Unterlagsplatte in einem Wasserbad, wobei die Oberseite der Unterlagsplatte mitsamt der darauf befindlichen getrockneten Wandfarbschicht aus dem Wasserbad herausragt;
- f- Entnehmen der Unterlagsplatte aus dem Wasserbad;
- g- Pressen und gleichzeitiges Trocknen der Wandfarbschicht;
- h- Abheben der getrockneten und gepressten Wandfarbschicht von der Unterlagsplatte mit einem Abhebewerkzeug.

Vorteilhaft wird für das erfindungsgemäße Verfahren eine saugfähige Unterlagsplatte verwendet, die durch Kontakt mit Wasser relativ rasch befeuchtet werden kann, um im befeuchteten Zustand eine zuvor bereits getrocknete Wandfarbschicht, die auf der Oberseite der Unterlagsplatte aufgetragen ist, leichter wieder von der Oberseite abnehmen zu können. Zweckmäßigerweise werden nach dem Trocknungsschritt -d- Umrisse von Konturen von Farbproben in die getrocknete Wandfarbschicht eingeritzt bzw. eingeschnitten. Alternativ dazu kann auch die gesamte aufgetragene Fläche der Wandfarbschicht als Farbprobekörper dienen.

Nach dem Einlegen der getrockneten Wandfarbschicht auf der Oberseite der Unterlagsplatte in einem Wasserbad lässt sich die angefeuchtete Wandfarbschicht spannungs- und beschädigungsfrei von der Unterlagsplatte abnehmen. Während des Einlegens im Wasserbad steht die Wandfarbschicht nicht direkt mit dem Wasser in Kontakt, sondern wird nur indirekt aufgrund des innerhalb der Unterlagsplatte aufsteigenden Feuchtespiegels angefeuchtet.

Zweckmäßig können dabei jene Teile bzw. Farbschichtreste entfernt werden, die außerhalb der Konturen der gewünschten Farbproben liegen.

Um während des Pressens und Trocknens der Wandfarbschicht ein unerwünschtes Verzerren bzw. Verwölben der Farbproben zu verhindern, können diese vorteilhaft mit einem saugfähigen sowie ebenen bzw. flachen Abdeckmaterial abgedeckt werden und anschließend zum Pressen beschwert werden.

Nach Austrocknung der Wandfarbschicht bzw. nach Erreichen der Massenkonstanz wird die Unterlagsplatte freigelegt und die gewünschten Schichtbereiche der Farbproben lassen sich im Anschluss spannungs- und deformationsfrei von der Unterlagsplatte entfernen. Somit erhält man mithilfe des erfindungsgemäßen Verfahrens freistehende, dünne Schichten aus Wandfarbe mit gewünschten Formen und Abmessungen, die als Farbprobekörper weiter getestet und untersucht werden können.

Besonders zweckmäßig kann es sein, wenn bei einem erfindungsgemäßen Verfahren vor dem Auftragen von Wandfarbe gemäß Schritt -b- die Oberseite der saugfähigen Unterlagsplatte mit Wasser befeuchtet wird. Durch das Befeuchten bzw. Besprühen der zu beschichtenden Oberseite der Unterlagsplatte mit Wasser wird verhindert, dass mögliche Lufteinlagerungen in der Unterlagsplatte während der weiteren Verfahrensschritte in die Wandfarbschicht diffundieren und zu unerwünschten Lufteinschlüssen in der Wandfarbschicht führen.

Vorteilhaft kann bei einem Verfahren gemäß der Erfindung nach dem Trocknen der Wandfarbschicht gemäß Schritt -d- zumindest ein Konturabschnitt einer Farbprobe mittels eines Schneidwerkzeugs in die getrocknete Wandfarbschicht eingeschnitten werden. Das Einschneiden von Konturen einer oder mehrerer Farbproben noch auf der Unterlagsplatte bietet den Vorteil, dass die Farbproben spannungs- und zerstörungsfrei bearbeitet werden können und die Unterlagsplatte Beschädigungen der Farbprobenkörper verhindert.

Zweckmäßig können bei einem erfindungsgemäßen Verfahren nach dem Entnehmen der Unterlagsplatte aus dem Wasserbad gemäß Schritt -f- sowie vor dem anschließenden Pressen und Trocknen gemäß Schritt -g- diejenigen Abschnitte der Wandfarbschicht, die als Farbreste außerhalb des zumindest einen freigeschnittenen Konturabschnitts einer Farbprobe liegen, von der Oberseite der Unterlagsplatte entfernt werden. Wie vorhin bereits dargestellt lassen sich in wiederangefeuchteter Lage die Farbreste der Wandfarbschicht, die außerhalb der gewünschten Farbproben liegen, besonders einfach von der Unterlagsplatte abheben.

Besonders vorteilhaft ist es, wenn bei einem erfindungsgemäßen Verfahren als saugfähige Unterlagsplatte eine Gipsplatte verwendet wird. Gips bietet aufgrund seiner hydrophilen Eigenschaften ein ausgezeichnetes Material, um als Unterlagsplatte im erfindungsgemäßen Verfahren zu dienen. Überdies ist Gips ein übliches Wandmaterial bzw. zumindest ein üblicher Inhaltsstoff von Wandmaterialien, auf denen Wandfarben üblicherweise aufgetragen werden. Die handelsüblichen Wandfarben, insbesondere Wandfarben zur Innenraumgestaltung, sind also bestens zum Auftragen auf einem Gips-Grund bzw. zumindest einem Gips enthaltenden Untergrund geeignet.

In einer weiteren vorteilhaften Ausführungsvariante des erfindungsgemäßen Verfahrens kann die Schichtstärke der aufgetragenen Wandfarbschicht so ausgewählt werden, dass diese nach dem Glätten gemäß Schritt -c- von 0,01 mm bis 1,5 mm, bevorzugt von 0,1 mm bis 1,0 mm, beträgt.

Besonders vorteilhaft kann es sein, wenn bei einem erfindungsgemäßen Verfahren für das Trocknen der Wandfarbschicht gemäß Schritt -d- Trocknungsbedingungen bei einer Trocknungstemperatur entsprechend der Raumtemperatur, bevorzugt von 20°C bis 25°C, sowie bei einer relativen Luftfeuchte von 40% bis 60%, bevorzugt von 45% bis 55%, ausgewählt werden, wobei vorzugsweise eine Trocknungsdauer bis zur Massenkonstanz der Wandfarbschicht gewählt wird. Diese Trocknungsbedingungen hinsichtlich der Trocknungstemperatur entsprechen den üblichen, von den Herstellern von Wandfarben meist angeführten "idealen" Trocknungsbedingungen, die bei Temperaturen von 23°C ± 2°C und einer relativen Luftfeuchte von 50% ± 5% nach ISO 554 liegen sollten.

Vorzugsweise erfolgt die Trocknung einer Probe einer Wandfarbschicht, bis Massenkonstanz erreicht ist und sich das Gewicht der aufgetragenen Farbprobe im Wesentlichen nicht mehr ändert.

Zweckmäßig kann in einer weiteren Variante des erfindungsgemäßen Verfahrens die saugfähige Unterlagsplatte im Wasserbad gemäß Schritt -e- bei einer Wassertemperatur entsprechend der Raumtemperatur, bevorzugt von 20°C bis 25°C, sowie während einer Dauer von 10 min bis 60 min, bevorzugt von 20 min bis 40 min, eingelegt werden, wobei die Unterlagsplatte vorzugsweise so gelagert wird, dass diese mit ihrer halben Plattendicke aus dem Wasserbad herausragt. Für übliche Beschichtungen im Bauwesen können beispielsweise 20 min als Lagerdauer im Wasserbad ausreichend sein.

Besonders vorteilhaft kann es sein, wenn beim erfindungsgemäßen Verfahren das Pressen und gleichzeitige Trocknen der Wandfarbschicht gemäß Schritt -g- bei einer Trocknungstemperatur entsprechend der Raumtemperatur, bevorzugt von 20°C bis 25°C, sowie während einer Trocknungsdauer von 1 Stunde bis 48 Stunden, bevorzugt von 12 Stunden bis 24 Stunden, durchgeführt wird.

In einer weiteren Ausführungsvariante des erfindungsgemäßen Verfahrens kann während des Pressens und gleichzeitigen Trocknens der Wandfarbschicht gemäß Schritt -g- die Wandfarbschicht mit einem saugfähigen Abdeckmaterial bedeckt werden, wobei vorzugsweise das Pressen bei einem Pressdruck von 20 kg/m2 bis 100 kg/m2, besonders bevorzugt bei einem Pressdruck von 50 kg/m2, durchgeführt wird.

Zweckmäßig dauert die Trocknung bis zum Erreichen einer ausreichenden Festigkeit der Wandfarbschicht, um eine schadensfreie Entfernung dieser Farbschicht vom Untergrund bzw. von der Unterlagsplatte zu gewährleisten. Der Pressdruck sollte dabei so gewählt werden, dass dieser der Wandfarbschicht nicht schadet.

In Vorversuchen wurde das erfindungsgemäße Verfahren bereits ausführlich getestet. Bei den bisher durchgeführten Versuchen kamen Gipsplatten als Unterlagsplatten mit einer beispielhaften Plattendicke von 30 mm und einer Dichte von rund 1000 kg/m³ zum Einsatz. Mithilfe des erfindungsgemäßen Verfahrens wurden damit Farbproben, die als längliche Zugproben konturiert sind, mit Form und Abmessungen gemäß DIN EN ISO 527-2 aus insgesamt 14 unterschiedlichen Wandfarben (5 witterungsbeständige Fassadenfarben für den Außeneinsatz und 9 verschiedene lnnenraumbeschichtungsfarben, darunter 2 Latexfarben) hergestellt. Dabei erfolgte das Auftragen der Wandfarbe auf den Gipsplatten jeweils mithilfe einer Schablone, um eine konstante Schichtstärke der geglätteten Wandfarbschicht von jeweils 0,40 mm auf der Gipsplatte zu erstellen.

Nach Austrocknen der Wandfarbe wurden die Umrisse der Zugproben ebenfalls mit einer Schablone auf der Wandfarbschicht angezeichnet und anschließend mit einem Skalpell in die Wandfarbschicht eingeschnitten. Danach wurde die Gipsplatte bis zur Hälfte der Plattendicke - somit etwa 15 mm tief - ins Wasser eines Wasserbads gelegt und verweilte darin etwa während einer Dauer von 20 min. Nach der Entnahme aus dem Wasser wurden die in der Schicht eingeschnittenen Farbproben in Form von Zugproben von der restlichen Wandfarbschicht befreit und zuerst mit zwei Papierblättern aus Saugpapier mit einer Grammatur von je 80 g/m² und zusätzlich mit zwei Papiertüchern abgedeckt. Zur Beschwerung kam dabei eine Marmorplatte als Presseinrichtung mit einer ebenen, glatten Oberfläche und einer Stärke von 20 mm zum Einsatz. Nach Austrocknen der Wandfarbschicht erfolgte das Entfernen der Farbproben bzw. Zugproben von der Gipsplatte mithilfe von Pinzetten und Spachteln. Die entfernten Farbproben wurden wieder mit Papier abgedeckt und mit der Marmorplatte beschwert. Nach Erreichen der Massenkonstanz waren die so erstellten Zugproben versuchsreif. Die Schichtstärke der trockenen Wandfarbschicht betrug dabei im Durchschnitt 0,28 mm.

Das erfindungsgemäße Verfahren ermöglicht durch den Einsatz eines mineralischen Untergrundes im Gegensatz zu dem in der Praxis angewandten Verfahren, bei dem die Wandfarbe auf einer Teflonfolie als Trägerfolie aufgetragen wird, die Erstellung freistehender Schichten aus Wandfarben jeglichen Typs. Durch das Auftragen der Wandfarbe auf einem mineralischen Untergrund, der in der Praxis üblich ist, wird eine materialkonforme Austrocknung der darauf erstellten Schicht ermöglicht. Somit lassen sich mit dem erfindungsgemäßen Verfahren freistehende Schichten erstellen, die nicht nur eben sind, sondern auch Schichtstärken und Qualitäten aufweisen, die in der Praxis vorkommen. Typische Schichtstärken handelsüblicher Wandfarben nach Verarbeitung an einer Wand- bzw. Deckenoberfläche bewegen sich üblicherweise von 0,1 mm bis 0,7 mm.

Des Weiteren lassen sich aus den mithilfe des erfindungsgemäßen Verfahrens ebene Wandfarbschichten bspw. Farbprobekörper herstellen, die eine Bestimmung der mechanischen Eigenschaften einer Wandfarbe, wie beispielsweise der Festigkeit und der Dehnfähigkeit, ermöglichen, ohne dabei die mechanischen Eigenschaften eines im Probekörper enthaltenen Trägermaterials berücksichtigen zu müssen. Somit werden bei der Auswertung der Versuche aufwändige Berechnungen oder Verfälschungen der ermittelten Messergebnisse aufgrund eines vorhandenen Trägermaterials vermieden.

Weitere Einzelheiten, Merkmale und Vorteile der Erfindung ergeben sich aus der nachfolgenden Erläuterung eines in den Zeichnungen schematisch dargestellten Ausführungsbeispiels für ein erfindungsgemäßes Verfahren. In den Zeichnungen zeigen:
- **Fig. 1** in einer isometrischen Ansicht schräg von vorne eine saugfähige Unterlagsplatte zur Durchführung des erfindungsgemäßen Verfahrens;
- **Fig. 2** in einer isometrischen Ansicht schräg von vorne die in Fig. 1 veranschaulichte Unterlagsplatte während des Auftragens von Wandfarbe;
- **Fig. 3** in einer isometrischen Ansicht schräg von vorne die in Fig. 2 dargestellte Anordnung während des Glättens der Wandfarbe bzw. der Herstellung einer gleichmäßigen Wandfarbschicht;
- **Fig. 4** in einer isometrischen Ansicht schräg von vorne das Ausschneiden von Farbproben aus der getrockneten Wandfarbschicht;
- **Fig. 5** in einer isometrischen Ansicht schräg von vorne die gemäß Fig. 4 ausgeschnittenen Farbproben in der getrockneten Wandfarbschicht;
- **Fig. 6** in einer Seitenansicht ein Wasserbad, in dem die Unterlagsplatte samt der darauf befindlichen getrockneten Wandfarbschicht eingelegt ist;
- **Fig. 7** in einer isometrischen Ansicht schräg von vorne während des Entfernens von jenen Abschnitten der Wandfarbschicht, die außerhalb der Konturen der Farbproben liegen;
- **Fig. 8** in einer Seitenansicht die verfahrensgemäße Anordnung während des Pressens und gleichzeitigen Trocknens der Wandfarbschicht;
- **Fig. 9** in einer isometrischen Ansicht schräg von vorne das Abheben von Farbproben der getrockneten und gepressten Wandfarbschicht von der Unterlagsplatte.

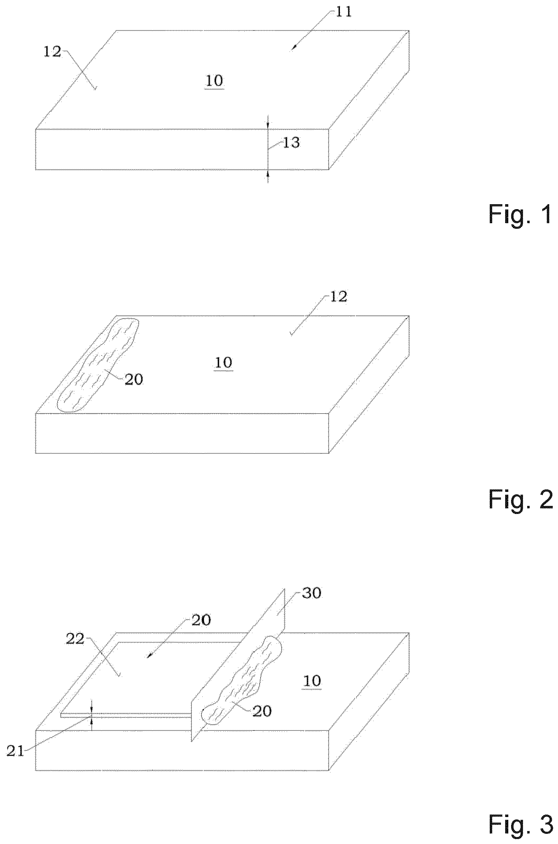

Im Detail zeigt **Fig. 1** eine saugfähige Unterlagsplatte 10, wobei hier eine Unterlagsplatte 10 aus Gips 11 verwendet wird. Die Unterlagsplatte 10 bzw. Gipsplatte 11 weist eine Oberseite 12 sowie eine Dicke 13 auf.

In **Fig. 2** ist bereits auf der Oberseite 12 der Unterlagsplatte 10 eine Menge an Wandfarbe 20 aufgebracht.

Wie in **Fig. 3** dargestellt wird anschließend diese Menge an Wandfarbe 20 unter Zuhilfenahme eines Glättwerkzeugs 30 geglättet und mit einer definierten Schichtstärke 21 als gleichmäßige Wandfarbschicht 22 auf der Oberseite 12 der Unterlagsplatte 10 aufgetragen. Vorteilhaft ist das Glättwerkzeug 30, hier beispielsweise eine Glättkelle, nach Art einer Schablone gearbeitet, wobei das Glättwerkzeug 30 an seinen Rändern auf der Unterlagsplatte 10 während des Glättvorgangs ansteht und mittig eine Ausnehmung entsprechend der gewünschten Schichtstärke 21 der Wandfarbschicht 22 aufweist.

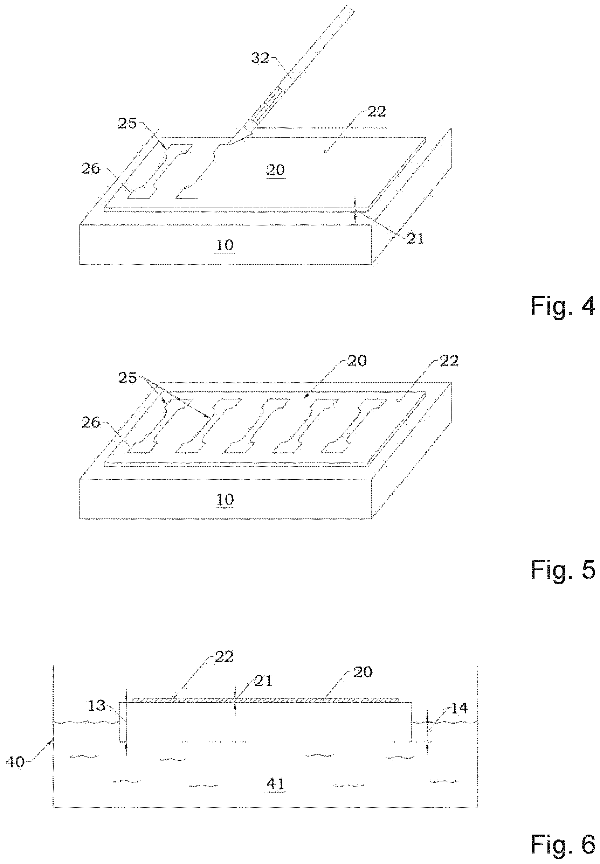

**Fig. 4** zeigt die Verfahrensanordnung während des Ausschneidens von Farbproben 25 aus der getrockneten Wandfarbschicht 22. Diese werden mit einem Schneidwerkzeug 32 wie beispielsweise einem Skalpell aus der getrockneten Wandfarbschicht 22 ausgeschnitten. Um standardisierte Farbproben 25 mit jeweils derselben Kontur 26 bzw. denselben Abmessungen zu erhalten, wird dazu zweckmäßig eine nicht explizit gezeigte Schablone entsprechend der jeweils gewünschten Kontur 26 verwendet.

**Fig. 5** zeigt die gemäß Fig. 4 ausgeschnittenen mehreren Farbproben 25 in der getrockneten Wandfarbschicht 22.

**Fig. 6** zeigt ein Wasserbad 40, in dem die Unterlagsplatte 10 samt der darauf befindlichen zuvor bereits getrockneten Wandfarbschicht 22, wie diese in Fig. 5 gezeigt wird, eingelegt ist. Die Unterlagsplatte 10 wird dabei so eingetaucht, dass die Unterlagsplatte 10 nur mit ihrer unteren halben Plattendicke 14 im Wasser eintaucht. Die Oberseite 12 der Unterlagsplatte 10 samt der darauf befindlichen Wandfarbschicht 22 ragen dabei aus dem Wasser. Da als Unterlagsplatte 10 ein saugfähiges Material, hier beispielsweise Gips 11, verwendet wird, steigt die Wasserfeuchte innerhalb der Unterlagsplatte 10 während der Einlegedauer der Unterlagsplatte 10 im Wasserbad 40 auf. Durch das Anfeuchten der Oberseite 12 der Unterlagsplatte 10 lässt sich die getrocknete Wandfarbschicht 22 vom feuchten Untergrund der Unterlagsplatte 10 leicht ablösen.

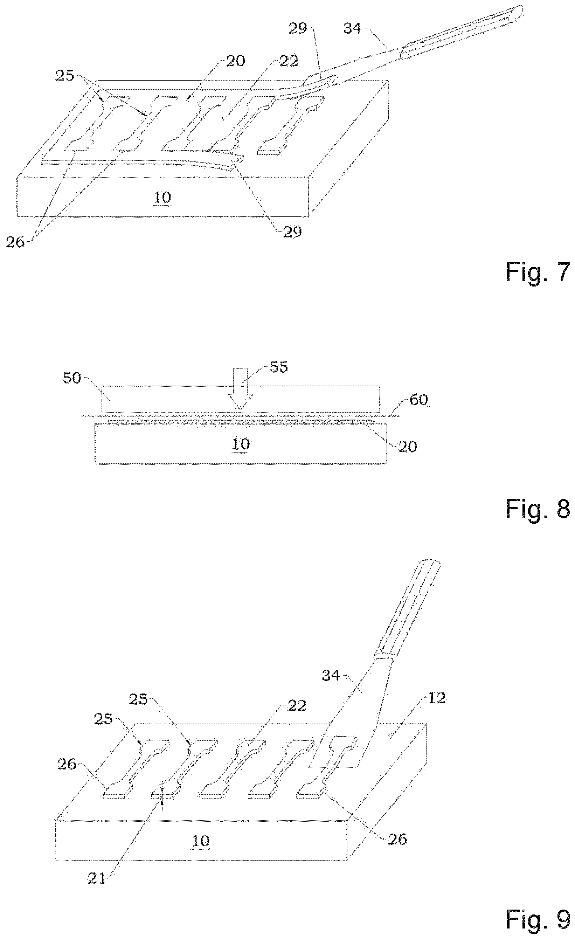

Wie in **Fig. 7** dargestellt, wird dieser Umstand zum Entfernen von jenen Abschnitten der Wandfarbschicht 22 genutzt, die als Farbschichtreste 29 außerhalb der Konturen 26 der Farbproben 25 liegen. Mittels eines Abhebewerkzeugs 34 wie beispielsweise einer Spachtel werden diese Farbschichtreste 29 entfernt, sodass nur mehr die zuvor ausgeschnittenen Farbproben 25 auf der Oberseite 12 der Unterlagsplatte 10 liegen bleiben.

**Fig. 8** zeigt die verfahrensgemäße Anordnung während des anschließenden Pressens und gleichzeitigen Trocknens der noch verbliebenen Farbproben 25 der Wandfarbschicht 22. Mit einer Presseinrichtung 50, beispielsweise einer schweren Steinplatte, wird eine durch den Pfeil 55 symbolisierte Presskraft 55 auf die Proben 25 gebracht. Damit die Farbproben 25 während des Pressens gleichzeitig auch trocknen können, wird zwischen die Oberseite der Wandfarbschicht 22 bzw. der Farbproben 25 und der Presseinrichtung 50 zuvor vor Beginn des Pressvorgangs noch ein saugfähiges Abdeckmaterial 60, beispielsweise ein Saugpapier, zwischengelegt. Während des Pressvorgangs nimmt das saugfähige Abdeckmaterial 60 bzw. Saugpapier noch Überschussfeuchte auf, die allenfalls in den Farbproben 25 noch vorhanden ist.

**Fig. 9** zeigt die verfahrensgemäße Anordnung nach dem Pressvorgang während des Abhebens von Farbproben 25 der getrockneten und gepressten Wandfarbschicht 22 von der Unterlagsplatte 10. Die fertig getrockneten und gepressten Farbproben 25 können mit einem Abhebewerkzeug 34 wie einer Spachtel von der Oberseite 12 der Unterlagsplatte 10 abgenommen werden, um diese Farbproben 25 anschließend beispielsweise für Materialprüfungsuntersuchungen verwenden zu können. Vorteilhaft weisen diese Farbproben 25 keine zusätzlichen Trägerschichten auf, sondern sind ausschließlich aus dem jeweiligen Wandfarbmaterial hergestellt.

### LISTE DER BEZUGSZEICHEN

- 10: Unterlagsplatte
- 11: Gips
- 12: Oberseite der Unterlagsplatte
- 13: Dicke der Unterlagsplatte
- 14: halbe Plattendicke
- 20: Wandfarbe
- 21: Schichtstärke der Wandfarbschicht
- 22: geglättete Wandfarbschicht
- 25: Farbprobe
- 26: Kontur einer Farbprobe
- 29: Farbschichtrest
- 30: Glättwerkzeug
- 32: Schneidwerkzeug
- 34: Abhebewerkzeug
- 40: Wasserbad
- 41: Wasser
- 50: Presseinrichtung
- 55: Presskraft (Pfeil)
- 60: Saugfähiges Abdeckmaterial

## Patentansprüche

1. Verfahren zur Herstellung einer freistehenden dünnen Schicht aus Wandfarbe, umfassend die folgenden Herstellungsschritte:
- a- Bereitstellen einer saugfähigen Unterlagsplatte (10) zum Auftrag von Wandfarbe (20);
- b- Auftragen von Wandfarbe (20) auf der Oberseite (12) der saugfähigen Unterlagsplatte (10);
- c- Glätten der auf der Unterlagsplatte (10) aufgetragenen Wandfarbe (20) mithilfe eines Glättwerkzeugs (30) zu einer gleichmäßigen Wandfarbschicht (22) mit einer definierten Schichtstärke (21);
- d- Trocknen der Wandfarbschicht (22) auf der Unterlagsplatte (10);
- e- Einlegen der saugfähigen Unterlagsplatte (10) in einem Wasserbad (40), wobei die Oberseite (12) der Unterlagsplatte (10) mitsamt der darauf befindlichen getrockneten Wandfarbschicht (22) aus dem Wasserbad (40) herausragt;
- f- Entnehmen der Unterlagsplatte (10) aus dem Wasserbad (40);
- g- Pressen (50) und gleichzeitiges Trocknen der Wandfarbschicht (22);
- h- Abheben der getrockneten und gepressten Wandfarbschicht (22) von der Unterlagsplatte (10) mit einem Abhebewerkzeug (34).

2. Verfahren nach Anspruch 1, **dadurch gekennzeichnet, dass** vor dem Auftragen von Wandfarbe (20) gemäß Schritt -b- die Oberseite (12) der saugfähigen Unterlagsplatte (10) mit Wasser befeuchtet wird.

3. Verfahren nach Anspruch 1 oder 2, **dadurch gekennzeichnet, dass** nach dem Trocknen der Wandfarbschicht (22) gemäß Schritt -d- zumindest ein Konturabschnitt (26) einer Farbprobe (25) mittels eines Schneidwerkzeugs (32) in die getrocknete Wandfarbschicht (22) eingeschnitten wird.

4. Verfahren nach Anspruch 3, **dadurch gekennzeichnet, dass** nach dem Entnehmen der Unterlagsplatte (10) aus dem Wasserbad (40) gemäß Schritt -f- sowie vor dem anschließenden Pressen (50) und Trocknen gemäß Schritt -g- diejenigen Abschnitte der Wandfarbschicht (22), die als Farbschichtreste (29) außerhalb des zumindest einen freigeschnittenen Konturabschnitts (26) einer Farbprobe (25) liegen, von der Oberseite (12) der Unterlagsplatte (10) entfernt werden.

5. Verfahren nach einem der Ansprüche 1 bis 4, **dadurch gekennzeichnet, dass** als saugfähige Unterlagsplatte (10) eine Gipsplatte (11) verwendet wird.

6. Verfahren nach einem der Ansprüche 1 bis 5, **dadurch gekennzeichnet, dass** die Schichtstärke (21) der aufgetragenen Wandfarbschicht (22) so ausgewählt wird, dass diese (21) nach dem Glätten gemäß Schritt -c- von 0,01 mm bis 1,5 mm, bevorzugt von 0,1 mm bis 1,0 mm, beträgt.

7. Verfahren nach einem der Ansprüche 1 bis 6, **dadurch gekennzeichnet, dass** für das Trocknen der Wandfarbschicht (22) gemäß Schritt -d- Trocknungsbedingungen bei einer Trocknungstemperatur entsprechend der Raumtemperatur, bevorzugt von 20°C bis 25°C, sowie bei einer relativen Luftfeuchte von 40% bis 60%, bevorzugt von 45% bis 55%, ausgewählt werden, wobei vorzugsweise eine Trocknungsdauer bis zur Massenkonstanz der Wandfarbschicht (22) gewählt wird.

8. Verfahren nach einem der Ansprüche 1 bis 7, **dadurch gekennzeichnet, dass** die saugfähige Unterlagsplatte (10) im Wasserbad (40) gemäß Schritt -e- bei einer Wassertemperatur entsprechend der Raumtemperatur, bevorzugt von 20°C bis 25°C, sowie während einer Dauer von 10 min bis 60 min, bevorzugt von 20 min bis 40 min, eingelegt wird, wobei die Unterlagsplatte (10) vorzugsweise so gelagert wird, dass diese mit ihrer halben Plattendicke (14) aus dem Wasserbad (40) herausragt.

9. Verfahren nach einem der Ansprüche 1 bis 8, **dadurch gekennzeichnet, dass** das Pressen (50) und gleichzeitiges Trocknen der Wandfarbschicht (22) gemäß Schritt -g-bei einer Trocknungstemperatur entsprechend der Raumtemperatur, bevorzugt von 20°C bis 25°C, sowie während einer Trocknungsdauer von 1 Stunde bis 48 Stunden, bevorzugt von 12 Stunden bis 24 Stunden, durchgeführt wird.

10. Verfahren nach einem der Ansprüche 1 bis 9, **dadurch gekennzeichnet, dass** während des Pressens (50) und gleichzeitigen Trocknens der Wandfarbschicht (22) gemäß Schritt -g- die Wandfarbschicht (22) mit einem saugfähigen Abdeckmaterial (60) bedeckt wird, wobei vorzugsweise das Pressen (50) bei einem Pressdruck (55) von 20 kg/m² bis 100 kg/m², besonders bevorzugt bei einem Pressdruck (55) von 50 kg/m², durchgeführt wird.
